# EUROPEAN PATENT APPLICATION

(11) **EP 4 268 839 A1**
(43) Date of publication of application: **01.11.2023**
(21) Application number: 21911555.7
(22) Date of filing: 22.12.2021
(51) Int. Cl.: A61K 38/00, A61P 1/16, C07K 14/47

(54) **PHARMACEUTICAL COMPOSITION FOR PREVENTING OR TREATING ALCOHOLIC AND NONALCOHOLIC FATTY LIVER DISEASE**

(30) Priority: 22.12.2020 KR 20200180640
(71) Applicant: MEDI & GENE, Seoul 02841 (KR)
(72) Inventor: SHIN, Min Jeong, Seoul 06285 (KR); JEONG, In Hyeok, Goyang-si Gyeonggi-do 10521 (KR); LEE, Ju Hee, Seoul 07524 (KR); LEE, Jin Young, Changwon-si Gyeongsangnam-do 51722 (KR)
(74) Representative: Vossius & Partner Patentanwälte Rechtsanwälte mbB
(86) International application number: PCT/KR2021/019660
(87) International publication number: WO 2022/139489

(57) **Abstract**

The present invention relates to a pharmaceutical composition for preventing or treating fatty liver disease, containing the ATPase inhibitory factor 1 (IF1) as an active ingredient, and the composition can be usefully used for the purpose of preventing and treating fatty liver disease, as the composition protects hepatocytes from toxicity due to alcohol; reduces the formation of fatty liver by suppressing lipid accumulation in liver tissue and an increase in the blood lipid concentration caused by excessive nutrition; suppresses liver fibrosis and inflammatory response; and increases the proportion of beneficial bacteria in the gut microbiome.

## Description

### [Technical Field]

The present invention relates to a pharmaceutical composition for preventing or treating alcoholic and non-alcoholic fatty liver disease, containing the ATPase inhibitory factor 1 (IF 1) as an active ingredient.

### [Background Art]

Along with the development of industry, Korean diets also tend to become westernized, and this, combined with the decline in physical activity of modern people, has led to an increase in the number of obese people, and the risk of various metabolic diseases caused by metabolic syndrome has been increased. Metabolic diseases cause various other diseases and are simultaneously becoming more and more serious as factors that adversely affect various biometabolisms in the human body such as aging, deterioration in immune system function, and stress.

Fatty liver disease is the most common liver disease, generally refers to excessive accumulation of fat in the liver, and indicates a state in which the neutral lipid content is 5% or more. Fatty liver disease may be classified as non-alcoholic fatty liver disease (NAFLD), whose main cause is metabolic syndromes such as diabetes, obesity and hyperlipidemia, and alcoholic fatty liver disease caused by alcohol intake, that is, excessive drinking.

Alcoholic fatty liver disease is a condition in which excessive fat accumulates in the liver due to lipogenesis promoted by continuous alcohol intake, and swollen hepatocytes caused by such conditions affect blood vessels or lymph glands, resulting in abnormal nutritional supply and immune function. Consequently, liver function deteriorates, and an imbalance in energy metabolism occurs. In particular, alcoholic fatty liver disease may develop into hepatitis or hepatic cirrhosis depending on the degree of alcohol intake, and thus causes a great risk to people's health and welfare

Non-alcoholic fatty liver disease is caused by other causes except for fatty liver disease caused by alcohol, and exhibits symptoms including inflammation, liver fibrosis, liver sclerosis, and the like. Non-alcoholic fatty liver disease may be divided as simple steatosis and non-alcoholic steatohepatitis (NASH), and NASH is a condition accompanied by hepatocyte damage and inflammation along with fat deposition, and may cause liver fibrosis, hepatic cirrhosis, and hepatocellular carcinoma.

Patients suffering from metabolic diseases such as obesity and diabetes have a high incidence of fatty liver (about 75% and 50%, respectively), and thus have received much attention, but since there are no precise onset mechanism and effective therapy and drugs, and only basic exercise and diet therapy have been proposed, there is a growing demand for a new therapeutic agent for fatty liver diseases.

### [Disclosure]

### [Technical Problem]

Under the circumstances described above, the present inventors conducted research to find a material which is safe for the human body and can treat fatty liver disease. As a result, the present inventors confirmed that IF1, which is a human-derived protein, protects hepatocytes from toxicity caused by alcohol, prevents lipid accumulation in liver tissue and an increase in the blood lipid concentration caused by excessive nutritional intake to reduce the formation of fatty liver, suppresses liver fibrosis and inflammatory response, and also increases the proportion of beneficial bacteria in the gut microbiome, and thus is effective for preventing and treating fatty liver disease, thereby completing the present invention.

Therefore, an object of the present invention is to provide a composition for preventing or treating alcoholic and non-alcoholic fatty liver diseases, including a human-derived protein, IF1, as an active ingredient.

### [Technical Solution]

To achieve the object, an aspect of the present invention provides a pharmaceutical composition for preventing or treating alcoholic and non-alcoholic fatty liver diseases, including IF1 as an active ingredient.

As used herein, alcoholic fatty liver disease refers to a condition in which fat is accumulated in hepatocytes due to excessive drinking, and when there are no particular symptoms in the early stage, but inflammation progresses, symptoms such as powerlessness, fatigue, fever, nausea, and vomiting appear. When alcoholic fatty liver disease is not properly treated, it progresses to alcoholic hepatic cirrhosis, and thus causes ascites, bleeding from esophageal varices, hepatic coma, and the like.

As used herein, non-alcoholic fatty liver disease (NAFLD) refers to a condition in which fat is accumulated in hepatocytes even though alcohol is not consumed or consumed in small amounts. Non-alcoholic fatty liver disease (NAFLD) is not a single disease, and includes various forms of liver diseases ranging from simple fatty liver which is not accompanied by inflammation to non-alcoholic steatohepatitis (NASH) and hepatic cirrhosis (liver cirrhosis).

As used herein, the term "prevention" refers to all actions that suppress the progression of alcoholic and non-alcoholic fatty liver diseases or delay the onset of the diseases by administering the pharmaceutical composition according to the present invention.

As used herein, the term "treatment" refers to all actions that ameliorate or beneficially change alcoholic and non-alcoholic fatty liver diseases by administering the pharmaceutical composition according to the present invention.

ATPase inhibitory factor 1 (IF1) is a major protein that is expressed intracellularly and binds to ATPase present in the mitochondrial membrane to interfere with rotational movement, and consequently, affects intracellular energy regulation and mitochondrial homeostasis by interfering with ATP synthesis/degradation by the electron transport chain. Although the action of IF1 is currently reported to have a positive effect on the amelioration of various diseases such as breast cancer and a degenerative nervous system disease, its effect on fatty liver disease has not been yet known.

In the present invention, the IF1 may be a protein including an amino acid sequence represented by SEQ ID NO: 1 or SEQ ID NO: 2. In an exemplary embodiment of the present invention, an IF1 sequence of SEQ ID NO: 1 was used.

**[Table 1]**

| **Name** | **Amino acid sequence** |
|---|---|
| Mouse IF 1 (SEQ ID NO: 1) | VSDSSDSMDT GAGSIREAGG AFGKREKAEE DRYFREKTKE QLAALRKHHE DEIDHHSKEI ERLQKQIERH KKKIQQLKNN H |
| Human IF1 (SEQ ID NO: 2) | GSDQSENVDR GAGSIREAGG AFGKREQAEE ERYFRAQSRE QLAALKKHHE EEIVHHKKEI ERLQKEIERH KQKIKMLKHD D |

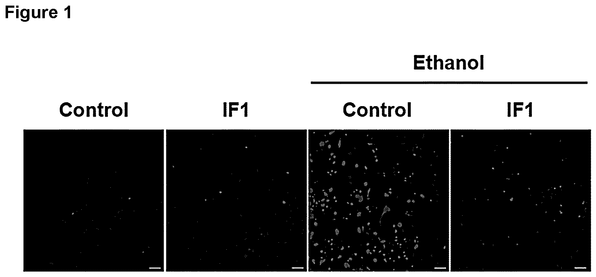

According to an exemplary embodiment of the present invention, when hepatocytes is pre-treated with IF1 and treated with alcohol, hepatocytes are protected because the production of reactive oxygen species is remarkably reduced compared to when hepatocytes are treated with alcohol alone (FIG. 1).

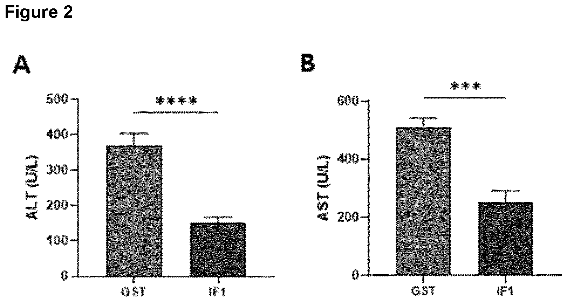

Further, since the activity of both ALT and AST, which are indicators of liver damage, is significantly reduced when IF1 is administered to mice in which non-alcoholic fatty liver disease was induced (FIG. 2), it can be seen that IF1 protects hepatocytes from liver damage caused by not only alcohol, but also lipid accumulation.

IF1 decreased blood total cholesterol and free fatty acid levels (FIG. 3), also decreased lipid accumulation in liver tissue (FIG. 4), and also suppressed excessive collagen accumulation in liver tissue and immune cell infiltration to ameliorate liver fibrosis and inflammatory response (FIG. 5), in mice in which non-alcoholic fatty liver disease was induced.

When simple fatty liver progresses to non-alcoholic steatohepatitis (NASH), hepatic fibrosis and inflammatory response appear, but the present invention is also useful for the treatment of non-alcoholic steatohepatitis because it ameliorates liver fibrosis and inflammatory response.

Therefore, since the pharmaceutical composition according to the present invention is characterized in that IF protects hepatocytes from toxicity caused by alcohol, reduces fatty liver formation, suppresses liver fibrosis and immune response, and increases the proportion of beneficial bacteria in the intestines, the pharmaceutical composition exhibits a therapeutic effect on both alcoholic and non-alcoholic fatty liver diseases, and may ameliorate symptoms that appear throughout the progression of non-alcoholic fatty liver disease.

The pharmaceutical composition of the present invention may be prepared in a form additionally including a suitable carrier, excipient or diluent which is typically used in the preparation of pharmaceutical compositions, and the carrier may include a non-naturally occurring carrier.

Specifically, the pharmaceutical composition may be used by being formulated in the form of an oral formulation such as a powder, a granule, a tablet, a capsule, a suspension, an emulsion, a syrup, and an aerosol, an external preparation, a suppository, and a sterile injection solution, according to a typical method.

In the present invention, examples of a carrier, an excipient and a diluent which may be included in the pharmaceutical composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When the pharmaceutical composition is prepared, the pharmaceutical composition is prepared using a diluent or excipient, such as a filler, an extender, a binder, a wetting agent, a disintegrant, and a surfactant, which are commonly used.

A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like. Further, in addition to a simple excipient, lubricants such as magnesium stearate and talc are also used.

A liquid preparation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, an aromatic, a preservative, and the like. Examples of a preparation for parenteral administration include an aqueous sterile solution, a non-aqueous solvent, a suspension, an emulsion, a freeze-dried preparation, and a suppository. As the non-aqueous solvent and the suspending agent, it is possible to use propylene glycol, polyethylene glycol, a vegetable oil such as olive oil, an injectable ester such as ethyl oleate, and the like.

The pharmaceutical compositions may be in the form of a sterile injectable preparation as a sterile injectable aqueous or oily suspension. The suspension may be formulated according to techniques known in the art using a suitable dispersant or wetting agent (for example, Tween 80) and a suspending agent. The sterile injectable preparation may also be a sterile injectable solution or suspension in a nontoxic parenterally acceptable diluent or solvent (for example, a solution in 1,3-butanediol). Examples of a vehicle and a solvent that may be acceptably used include mannitol, water, Ringer's solution, and an isotonic sodium chloride solution. Furthermore, sterile, non-volatile oils are typically used as a solvent or suspending medium. Any mild or non-volatile oil, including synthetic mono- or diglycerides, may be used for this purpose. Natural oils in which fatty acids such as oleic acid and glyceride derivatives thereof are pharmaceutically acceptable (for example, olive oil or castor oil), particularly polyoxyethylated versions thereof, are also useful in injectable preparations.

Parenteral administration of the pharmaceutical composition according to the present invention is particularly useful when a target treatment is associated with sites or organs readily accessible by topical application. Examples of a carrier for topical administration of the compound of the present invention include, but are not limited to, mineral oil, liquid paraffin, white petrolatum, propylene glycol, polyoxyethylene, polyoxypropylene compounds, emulsifying wax and water.

The content of the active ingredient included in the pharmaceutical composition of the present invention is not particularly limited, but the active ingredient may be included in a content of 0.0001 to 50 wt%, more preferably 0.01 to 10 wt% based on the total weight of a final composition.

Another aspect of the present invention provides a method for treating alcoholic and non-alcoholic fatty liver diseases, the method including administering the composition for preventing or treating alcoholic and non-alcoholic fatty liver diseases to an individual in need of treatment.

Since the above-described pharmaceutical composition for preventing or treating alcoholic and non-alcoholic fatty liver diseases is used in the treatment method of the present invention, the description of the content common between the two will be omitted in order to avoid the excessive complexity of the present specification.

The method for administering the pharmaceutical composition may be oral or parenteral as described above, and the dosage of the pharmaceutical composition may be determined by a therapeutic expert.

### [Advantageous Effects]

IF1, which is a human-derived protein, protects hepatocytes from toxicity caused by alcohol, suppresses lipid accumulation in liver tissue and an increase in the blood lipid concentration caused by excessive nutritional intake to reduce the formation of fatty liver, suppresses liver fibrosis and inflammatory response, and increases the proportion of beneficial bacteria in the gut microbiome, and thus can be usefully used for the purpose of preventing and treating fatty liver disease.

### [Description of Drawings]

FIG. 1 shows the results of confirming the toxicity-protective effect of treatment of hepatic cell lines with IF1 as the effect of suppressing the production of reactive oxygen species (ROS).
FIG. 2 shows the results of confirming changes in blood liver enzyme values after the administration of IF1 to fatty liver disease-induced mice: measurement results of aspartate aminotransferase (A-ALT); and alanine aminotransferase (B-AST).

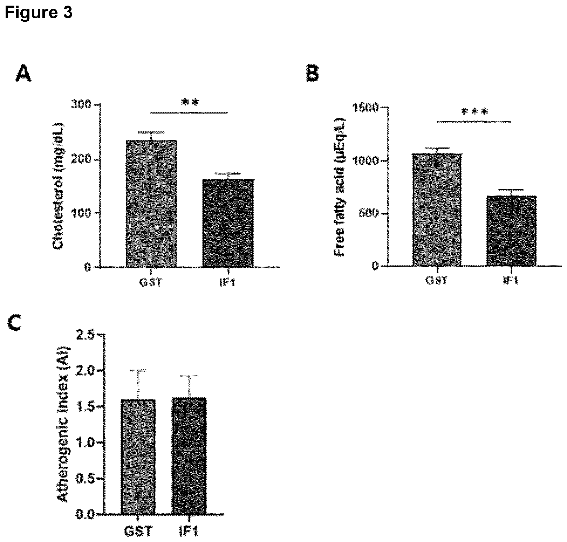

FIG. 3 shows the results of confirming changes in blood lipid values after the administration of IF1 to fatty liver disease-induced mice: A-cholesterol; B-free fatty acid (FFA); and C-atherogenic index

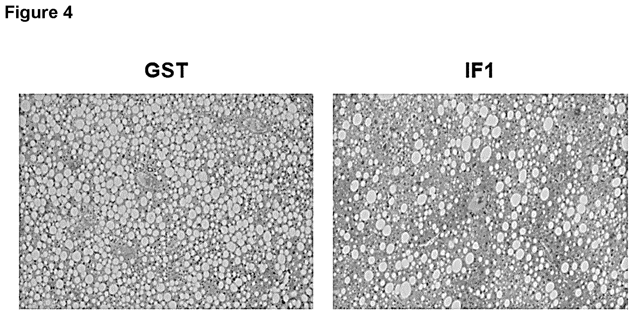

FIG. 4 shows the results of confirming the degree of fat accumulation in liver tissue after the administration of IF1 to fatty liver disease-induced mice.

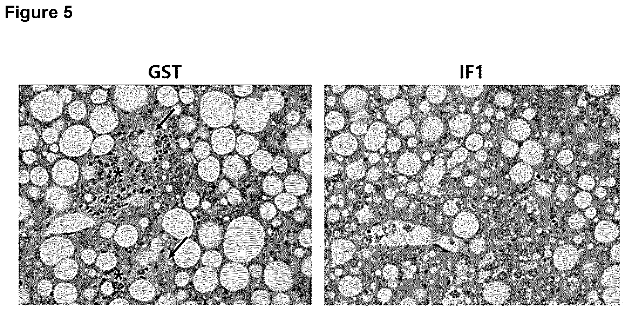

FIG. 5 shows the results of confirming the degree of amelioration of liver fibrosis and inflammatory response in liver tissue after the administration of IF1 to fatty liver disease-induced mice.

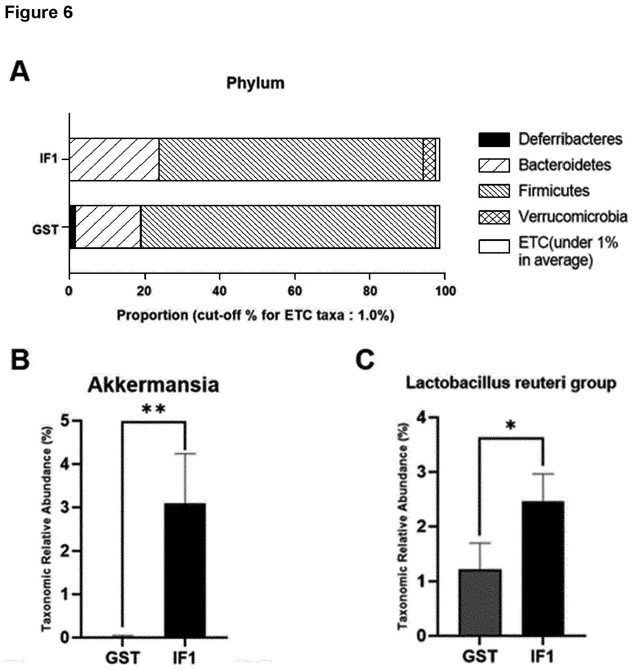

FIG. 6 shows the results of confirming changes in the gut microbiome after the administration of IF1 to fatty liver disease-induced mice: A-changes in the proportion of the gut microbiome; B-*Akkermansia muciniphila* whose proportion is specifically changed by IF; and *Lactobacillus reuteri* whose proportion is specifically changed by C-IF.

### [Modes of the Invention]

Hereinafter, one or more specific exemplary embodiments will be described in more detail through examples. However, these examples are provided only for exemplarily explaining the one or more specific exemplary embodiments, and the scope of the present invention is not limited to these examples.

### Example 1: Confirmation of protective effect of IF1 against alcohol toxicity in hepatocytes

In order to investigate the protective effect of IF1 against alcohol toxicity, hepatocytes were treated with alcohol, which is a representative material that shows toxicity, and the degree of production of reactive oxygen species depending on the treatment with IF was measured. It is known that alcohol promotes the production of reactive oxygen species in the metabolic process, and the produced reactive oxygen species act as a toxin to induce cell damage and simultaneously accelerate the inflammatory response and fibrosis process in liver tissue.

An HepG2 hepatocyte line was inoculated into 8-well chamber slides, cultured in an incubator at 37°C with 5% CO₂, and pretreated with a control material (PBS) and IF1 for 6 hours. Thereafter, the hepatocyte line was treated with 1 M ethanol for 18 hours to induce the production of reactive oxygen species. The cells were washed twice with HBSS, and then treated with 25 µM dichlorodihydrofluorescein diacetate (DCF-DA) at 37°C for 1 hour to stain reactive oxygen species. When the staining was completed, the cells were washed twice with HBSS and observed under a confocal microscope while soaked in an HBSS buffer containing 25 mM HEPES.

As a result, it could be seen that IF1 suppresses the production of reactive oxygen species by alcohol in hepatocytes by confirming that fluorescence intensity is weak in an experimental group pretreated with IF1, and then treated with alcohol compared to an experimental group treated with alcohol alone (FIG. 1).

### Example 2: Confirmation of fatty liver ameliorating effect by IF1 administration in non-alcoholic fatty liver disease-induced mice

### 2-1. Construction of non-alcoholic fatty liver disease-induced mouse model

Five-week-old C57BL/6J male mice were obtained from Orient Bio (Seongnam, Korea) and subjected to a one-week adaptation period, and then fatty liver was induced. All mice were kept at 18 to 24°C and 50 to 60% humidity as a breeding environment throughout the experiment, and fed ad libitum during both the adaptation period and the fatty liver induction period.

After a one-week adaptation period was completed, mice were fed a D09100310 product from Research Diet for 34 weeks to induce non-alcoholic fatty liver disease. Detailed ingredients of the product are shown in the following Table 2.

**[Table 2]**

| |
|---|
| 40 kcal% fat (palm oil) |
| 20 kcal% fructose |
| 2% cholesterol |

### 2-2: Administration of IF1 to non-alcoholic fatty liver-induced mice

The mice in which non-alcoholic fatty liver was induced were divided into two groups, and each group was bred under the following conditions for 9 weeks. For the IF1 protein, an IF1 coding sequence including GST-tag was cloned into E. *coli*, a recombinant protein was isolated and purified, and the GST-tag protein was used as a control.
Control: GST protein 7.5 mg/kg BW intraperitoneal injection, n=7
Experimental group: IF1 protein 7.5 mg/kg BW intraperitoneal injection, n=9

### 2-3: Analysis of liver enzyme values (ALT/AST) and lipid concentration

At week 43 after all the experiments were terminated, mice were sacrificed after measuring their body weights. Specifically, after the mice were anesthetized, blood was collected with an insulin syringe, and the collected blood was immediately centrifuged at 2,500 rpm for 30 minutes at 4°C to isolate/obtain serum. Thereafter, the remaining tissue was separated to measure mass and length, fixed in a 10% formaldehyde solution and stored at -80°C for subsequent analysis.

At the end of the experiment, the body weight of the control (GST administration group) and the experimental group (IF 1 administration group) was shown to be 45.8 ± 3.9 g and 42.4 ± 2.6 g, respectively, so the body weight in the experimental group was reduced by about 7.5%, and the weight of the liver tissue in the control (GST administration group) and the experimental group (IF 1 administration group) was shown to be 3.12 ± 0.5 g and 2.65 ± 0.6 g, respectively, so it was observed that the weight of the liver tissue in the experimental group was reduced by about 1.5%.

As a result of measuring liver injury index enzyme activity (ALT/AST) in the collected serum, it was confirmed that both serum ALT and AST were significantly reduced in the experimental group (IF 1 administration group) compared to the control group (GST administration group) (FIG. 2).

Further, as a result of analyzing blood lipid concentrations, both total cholesterol and free fatty acid were significantly reduced in the experimental group (IF 1 administration group). Since the atherogenic index (AI) (low-density lipoprotein cholesterol (LDLc)/high-density lipoprotein cholesterol (HDLc)), which is used as a cardiovascular biomarker, was 1.60±0.4/1.63±0.3, it was confirmed that there is no significant difference between the control and the experimental group (FIG. 3).

### 2-4. Analysis of liver tissue change

The structure of the mouse liver tissue obtained in Example 2-3 was analyzed by haematoxylin and eosin (H&E) staining and Masson's trichrome staining. Since H&E stains the nucleus in blue and the extracellular matrix and intracellular structures in red, it was used to analyze cell structure. Since Masson's trichrome staining stains collagen fibers in blue, the cytoplasm in red, and cell nuclei in blackish brown, it was used to analyze the degree of collagen accumulation, which is responsible for liver fibrosis induced by fatty liver disease, and inflammatory-cell infiltration, which is responsible for inflammation.

As a result of staining, it could be confirmed that lipid accumulation in the liver tissue was greatly reduced in the experimental group (IF 1 administration group) compared to the control (GST administration group) (FIG. 4). Furthermore, by confirming through Masson's trichrome staining that excessive accumulation of collagen (arrow) and immune cell infiltration phenomenon (asterisk) appearing in the control (GST administration group) were remarkably reduced to 19% in the experimental group (IF 1 administration group), it could be seen that IF1 ameliorates liver fibrosis and inflammatory response (FIG. 5).

### 2-5. Analysis of liver fibrosis index

Changes in expression of collagen type 1 alpha 1 (collagen 1a1, Col1a1), collagen type 5 alpha 1 (Col5a1), periostin and lysyl oxidase (LOX) genes, which are major biomarkers of liver fibrosis, were confirmed.

As a result of confirmation, by confirming that the expression of liver fibrosis biomarkers decreased in the experimental group (IF1 administration group) compared to the control (GST administration group), it could be seen that IF1 ameliorates liver fibrosis by regulating liver fibrosis at the gene level (Table 3).

**[Table 3]**

| Gene to be analyzed | Gene expression ratio (compared to control, %) |
|---|---|
| col1a1 | 77.9 |
| col5a1 | 42.9 |
| Periostin | 87.5 |
| LOX | 71.3 |

To additionally verify the effect of IF1, Gal-3 was specifically stained to observe and quantify the degree of expression. As a result, the liver fibrosis ameliorating effect of IF1 was verified at the molecular level by confirming that the accumulation of collagen type 1 alpha 1 (Col1a1) and galectins (galetin-3, Gal-3) was significantly reduced by 13% and 12%, respectively, in the experimental group (IF 1 administration group) compared to the control (GST administration group).

### 2-6. Analysis of gut microbiota change

The change and functional maintenance of the gut microbiome are widely known to play a major role in metabolic diseases including non-alcoholic fatty liver. In particular, since the gut microbiota affects the development and fibrosis of non-alcoholic fatty liver, the effect on this was confirmed.

Feces of mice in which fatty liver was induced by the method of Example 2-2 were collected, and genomic DNA was extracted to obtain 16s rRNA of the gut microbiota. For the obtained genetic information, the composition ratio and degree of variation of each subgroup for the microbiome were observed using next generation sequencing (NGS).

As a result, it could be confirmed that in the control (GST administration group) and the experimental group (IF1 administration group), the proportions of Deferribacteres, Bacteroidetes, Firmicutes and Verrucomicrobia were changed (FIG. 6A). Among the changes in the entire gut microbiome, the microorganisms that notably increased were *Akkermansia muciniphila* and *Lactobacillus reuteri* which are beneficial gut bacteria (FIGS. 6B and 6C). *Akkermansia muciniphila* is a representative beneficial bacterium that eats the muscin layer in the intestine and secretes short-chain fatty acids (SCFAs) to reduce inflammation and ameliorate metabolic syndrome or obesity. *Lactobacillus reuteri* is a beneficial bacterium that produces lactic acid to exhibit antibacterial effects.

## Claims

1. A pharmaceutical composition for use in preventing or treating alcoholic and non-alcoholic fatty liver disease, containing the ATPase inhibitory factor 1 (IF1) as an active ingredient.

2. The pharmaceutical composition of claim 1, wherein the composition protects hepatocytes from toxicity caused by alcohol.

3. The pharmaceutical composition of claim 1, wherein the composition suppresses an increase in blood lipid concentration.

4. The pharmaceutical composition of claim 1, wherein the composition suppresses lipid accumulation in liver tissue.

5. The pharmaceutical composition of claim 1, wherein the composition suppresses liver fibrosis.

6. The pharmaceutical composition of claim 1, further comprising a pharmaceutically acceptable carrier, excipient, or diluent.

7. A method for treating alcoholic and non-alcoholic fatty liver disease, the method comprising administering the composition of claim 1 to an individual in need of treatment.

8. The method of claim 7, wherein the composition protects hepatocytes from toxicity caused by alcohol.

9. The method of claim 7, wherein the composition suppresses an increase in blood lipid concentration.

10. The method of claim 7, wherein the composition suppresses lipid accumulation in liver tissue.

11. The method of claim 7, wherein the composition suppresses liver fibrosis.
